## Europäisches Patentamt

## European Patent Office

## Office européen des brevets

(19)

(11) Veröffentlichungsnummer: **0 167 141**

**B1**

(12) # EUROPÄISCHE PATENTSCHRIFT

(45) Veröffentlichungstag der Patentschrift:
**29.04.87**

(51) Int. Cl.⁴: **C 07 C 120/08, C 07 C 121/26**

(21) Anmeldenummer: **85108153.9**

(22) Anmeldetag: **01.07.85**

(54) **Verfahren zur Herstellung von Adipodinitril.**

(30) Priorität: **05.07.84 DE 3424701**

(43) Veröffentlichungstag der Anmeldung:
**08.01.86 Patentblatt 86/2**

(45) Bekanntmachung des Hinweises auf die Patenterteilung:
**29.04.87 Patentblatt 87/18**

(84) Benannte Vertragsstaaten:
**BE DE FR GB IT NL**

(56) Entgegenhaltungen:
**US-A-3 299 116**

**HOUBEN-WEYL: "Methoden der Organischen Chemie", vierte Auflage, Band VIII, Sauerstoffverbindungen III, 1952, Seite 330, Georg Thieme Verlag, Stuttgart; "Herstellung von Nitrilen durch Wasserabspaltung aus Carbonsäureamiden"**

(73) Patentinhaber: **BASF Aktiengesellschaft, Carl-Bosch- Strasse 38, D-6700 Ludwigshafen (DE)**

(72) Erfinder: **Dockner, Toni, Dr., In der Grossgasse 6, D-6701 Meckenheim (DE)**
Erfinder: **Sauerwald, Manfred, Dr., Gebhardstrasse 16, D-6701 Roedersheim- Gronau (DE)**
Erfinder: **Manegold, Jost Henrich, Dr., Neustadter Strasse 29, D-6715 Lambsheim (DE)**
Erfinder: **Leitner, Hans, Dr., Taunusstrasse 22, D-6710 Flomersheim (DE)**

## Beschreibung

Adipodinitril wird in großemechnischen Umfang durch Umsetzen von Adidimsäure mit Ammoniak hergestellt. Nach einem in der US-PS 3 153 084 beschriebenen Verfahren setzt man Adipinsäure und Ammoniak in der Gasphase an einem gesinterten Borsäure/Phosphorsäurekatalysator zu Adipodinitril um. Die Arbeitsweise in der Gasphase hat jedoch den Nachteil, daß der Katalysator rasch durch Belegung mit Zersetzungsprodukten inaktiviert wird und zudem erhebliche Mengen an schwer abtrennbaren Nebenprodukten wie 2-Cyanocyclopenten-1-yl-amin und 2-Cyano-cyclopentanon entstehen. Nach einem anderen in der GB-PS 5 37 954 beschriebenen Verfahren wird geschmolzene Adipinsäure mit Ammoniak in Gegenwart von sauren Katalysatoren wie Phosphorsäure umgesetzt. Hierbei erhält man Adipodinitril jedoch nur in mäßigen Ausbeuten. Aus der US-PS 3 299 116 ist auch schon ein Verfahren zur Herstellung von Adipodinitril bekannt, bei dem man Adipinsäure gelöst in Adipodinitril als Verdünnungsmittel mit Ammoniak in Gegenwart von sauren Katalysatoren wie Phosphorsäure umsetzt. Dieses Verfahren hat jedoch den Nachteil, daß man das als Verdünnungsmittel verwendete Adipodinitril in relativ großen Mengen im Kreis führen muß. Hierbei ist es erforderlich, Adipodinitril jeweils vom Katalysator und den Crackprodukten abzutrennen und wieder zurückzuführen. Darüber hinaus wird Adipodinitril mehrmals den schädigenden hohen Temperaturen ausgesetzt, wobei es zur Bildung von unerwünschten Nebenprodukten kommt.

Es war deshalb die technische Aufgabe gestellt, die Herstellung von Adipodinitril durch Umsetzen von Adipinsäure und Ammoniak in flüssiger Phase durchzuführen, wobei man Adipodinitril nur kurzzeitig den schädigenden hohen Reaktionstemperaturen aussetzt, der Katalysator nicht fortlaufend abgetrennt werden muß, die Bildung von unerwünschten Nebenprodukten vermindert wird und zudem die Crackprodukte von den Wertprodukten getrennt werden und im Verdünnungsmittel verbleiben.

Diese technische Aufgabe wird gelöst in einem Verfahren zur Herstellung von Adipodinitril durch Umsetzen von Adipinsäure mit überschüssigem Ammoniak bei einer Temperatur von 250 bis 350° C in flüssiger Phase unter Mitverwendung von Verdünnungsmitteln, dadurch gekennzeichnet, daß man die Umsetzung unter Mitverwendung von unter Reaktionsbedingungen inerten flüssigen Kohlenwasserstoffen als Verdünnungsmittel durchführt.

Das neue Verfahren hat den Vorteil, daß keine großen Mengen an Verdünnungsmitteln im Kreis geführt werden müssen. Weiter hat das neue Verfahren den Vorteil, daß Adipodinitril nur kurzzeitig den hohen Reaktionstemperaturen ausgesetzt wird. Ferner hat das neue Verfahren den Vorteil, daß bei Mitverwendung von Katalysatoren diese im Verdünnungsmittel verbleiben können. Schließlich hat das neue Verfahren den Vorteil, daß weniger unerwünschte Nebenprodukte gebildet werden und zudem die Reinigung des Adipodinitrils vereinfacht wird, da die Crackprodukte im Verdünnungsmittel verbleiben.

Erfindungsgemäß geht man von Adipinsäure aus, die man zweckmäßig in geschmolzener Form z.B. mit einer Temperatur vom 160 bis 180° C der Reaktion zuführt. Adipinsäure wird mit überschüssigem Ammoniak vorteilhaft in einer Menge von 3 bis 15 Mol, insbesondere 5 bis 10 Mol je Mol Adipinsäure umgesetzt.

Erfindungsgemäß wird die Umsetzung in unter Reaktionsbedingungen inerten, flüssigen Kohlenwasserstoffen durchgeführt. Vorzugsweise werden hochsiedende Mineralölfraktionen verwendet. Geeignete Verdünnungsmittel sind beispielsweise Vakuumgasöl, Vakuumrückstand, Heizöl S, geschmolzenes Paraffinwachs oder aromatisches Kohlenwasserstofföl. Vorteilhaft haben die Verdünnungsmittel einen Siedepunkt von mindestens 350° C, insbesondere von 400 bis 550° C.

Die Umsetzung wird bei einer Temperatur von 250 bis 350° C, insbesondere bei einer Temperatur von 280 bis 330° C durchgeführt. Im allgemeinen hält man bei der Umsetzung Atmosphärendruck ein, doch ist es möglich die Umsetzung auch unter geringem Überdruck oder Unterdruck durchzuführen.

Vorteilhaft wird die Umsetzung unter Mitverwendung von Katalysatoren durchgeführt. Geeignete Katalysatoren sind beispielsweise Fhosphorsäure, Phosphorsäure auf einem Träger wie Siliciumdioxid, Phosphorsäureester vorzugsweise hochsiedende Phosphorsäureester insbesondere hochsiedende Monoester der Phosphorsäure wie Phosphorsäure-mono-p-nonylphenylester oder Ammoniumsalze der Phosphorsäure wie Mono-(N-methylditridecylammonium)-dihydrogemphosphat. Unlösliche Katalysatoren werden zweckmäßig im Verdünnungsmittel in suspendierter Form verwendet. Die Katalysatoren werden vorteilhaft dem Verdünnungsmittel in einer Menge von 0,01 bis 25 Gew.-%, vorzugsweise 0,1 bis 10 Gew.-%, insbesondere 0,5 bis 5 Gew.-% zugesetzt.

Schwerflüchtige Nebenprodukte wie Polymere und teerige Zersetzungprodukte verbleiben in suspendierter oder gelöster Form im Verdünnungsmittel und können mit diesem kontinuierlich oder diskontinuierlich ausgeschleust werden. Es ist deshalb zweckmäßig, einen Teil z.B. je Stunde 0,1 bis 10 Gew.-%, des Verdünnungsmittels auszutauschen. Vorteilhaft wird das ausgeschleuste Mineralöl zur Energiegewinnung unterfeuert. Es versteht sich, daß man die ausgeschleuste Menge an Verdünnungsmittel und ggf. Katalysator ersetzt. Vorzugsweise wird Adipodinitril in dem Maße wie es entsteht in der Regel zusammen mit Zwischenverbindungen aus dem Reaktionsgemisch ausgetragen.

Mit dem überschüssigen Ammoniak werden z.B. aus dem flüssigen Reaktionsgemisch Adipodinitril sowie Zwischenverbindungen, wie Cyanvaleriansäure und Cyanvaleriansäureamid sowie Wasser, Kohlendioxid sowie flüchtige Nebenprodukte ausgetragen. Der gasförmige Austrag wird zweckmäßig abgekühlt und einer Destillationskolonne zugeführt. Über Kopf werden Ammoniak, Wasser sowie geringe Mengen Kohlendioxid, Cyclopentanon und Cyclopentanonderivate abgezogen. Ammoniak wird abgetrennt und in die Reaktion zurückgeführt. Als mittlere Fraktion wird technisch reines Adipodinitril erhalten. Am Boden der Kolonne verbleiben Zwischenverbindungen wie Cyanvaleriansäure und Cyanvaleriansäureamid, die ebenfalls wieder der Reaktion zugeführt werden.

Die Umsetzung führt man beispielsweise in Rührkesseln oder zylindrischen Reaktoren oder Füllkörperkolonnen durch. Diese sind zweckmäßig mit dem als Verdünnungsmittel verwendeten inerten Kohlenwasserstoff, der ggf. Katalysatoren enthält, z.B. bis zu 2/3, beschickt. Schmelzflüssige Adipinsäure und gasförmiges Ammoniak werden vorteilhaft von unten kontinuierlich zugeführt, wobei man vorzugsweise je Liter Reaktionsvolumen (Verdünnungsmittel) 0,1 bis 2 kg Adipinsäure je Stunde zuführt. Das flüssige Reaktionsgemisch wird auf der vorgenannten Temperatur gehalten und die mit dem überschüssigen Ammoniak ausgetragenen Produkte nach Kondensieren durch Destillation getrennt. Der Nebenprodukte enthaltende inerte Kohlenwasserstoff wird fortlaufend partiell abgezogen und durch frischen inerten Kohlenwasserstoff ersetzt.

Das Verfahren nach der Erfindung sei in folgenden Beispielen veranschaulicht:

## Beispiel 1

Ein Rührkessel von 2 l Inhalt wird mit 1000 g Vakuumrückstand (KP 400° C), der 1 Gew.-% Phosphorsäure enthält, beschickt. Der Vakuumrückstand wird bei einer Temperatur von 300° C gehalten und von unten stündlich 100 g schmelzflüssige Adipinsäure (160° C)und 150 Nl Ammoniak zugeführt. Die den Rührkessel verlassenden gasförmigen Produkte werden abgekühlt und destilliert. Überschüssiges Ammoniak wird im Kreis geführt. Nach einer Reaktionszeit von 5 Stunden werden 225,1 g technisch reines Adipodinitril und 150 g eines Rückstandes erhalten, der im wesentlichen aus Zwischenprodukten besteht. Letztere werden erneut der Reaktion zugeführt, wobei man weitere 114,7 g Adipodinitril erhält. Dies entspricht einer Gesamtausbeute vom 91,8 %.

## Beispiel 2

Man verfährt wie im Beispiel 1 beschrieben, verwendet jedoch 10 Gew.% eines Katalysators aus Siliciumdioxid und Phosphorsäure, der 10 Gew.-% Phosphorsäure auf Siliciumdioxid enthält. Man führt stündlich 300 g aufgeschmolzene Adipinsäure und 370 Nl Ammoniak zu. Die übrigen Reaktionsbedingungen entsprechen denjenigen des Beispiels I. M.an erhält nach einer Reaktionszeit won 5 Stunden 723,7 g technisch reines Adipodinitril. 310 g eines im wesentlichen aus Zwischenprodukten bestehenden Destillationsrückstands werden erneut umgesetzt und nochmals 229,6 g Adipodinitril erhalten. Dies entspricht einer Gesamtausbeute von 85,9 %.

## Beispiel 3

Ein beheizbares Rohr mit einem inneren Durchmesser von 60 mm und einer Länge von 1500 mm wird mit 1000 g Vakuumrückstand gefüllt und auf 300° C. erhitzt. Von unten werden stündlich 100 g geschmolzene Adipinsäure und 150 Nl Ammoniak zugeführt. Am Kopf des Rohres werden die gasförmigen Nebenprodukte abgezogen, kondensiert und getrennt. Überschüssiges Ammoniak wird wieder der Reaktion zugeführt. Nach einer Reaktionszeit von 5 Stunden erhält man unter Rückführen der erhaltenen Zwischenprodukte insgesamt 251,6 g technisch reines Adipodinitril. Die Ausbeute beträgt 68,0 %.

## Beispiel 4

Der im Beispiel 3 beschriebene Reaktor wird mit 1700 g Vakuumrückstand gefüllt, der 1 Gew.-% Phosphorsäure enthält. Bei einer Temperatur von 300° C werden von unten stündlich 1000 g geschmolzene Adipinsäure und 1500 Nl Ammoniak eingeleitet. Das den Reaktor verlassende gasförmige Reaktionsgemisch wird abgekühlt und destilliert. Überschüssiges Ammoniak sowie der Destillationsrückstand, der aus Zwischenprodukten besteht, wird zurückgeführt. Als mittlere Fraktion werden stündlich 705 g Adipodinitril erhalten. Dies entspricht einer Ausbeute von 95,3 %. Zudem werden stündlich 50 g des Reaktorinhalts abgelassen und durch frischen Katalysator enthaltenden Vakuumrückstand ersetzt.

## Patentansprüche

1. Verfahren zur Herstellung von Adipodinitril durch Umsetzen von Adipinsäure mit überschüßigem Ammoniak bei einer Temperatur von 250 bis 350°C in flüssiger Phase unter Mitverwendung von Verdünnungsmitteln, dadurch gekennzeichnet, daß man unter Reaktionsbedingungen flüssige inerte Kohlenwasserstoffe als Verdünnungsmittel verwendet.

2. Verfahren nach Amspruch 1, dadurch gekennzeichnet, daß man Mineralöle mit einem Siedepunkt von mindestens 350°C verwendet.

3. Verfahren nach den Ansprüchen 1 und 2, dadurch gekennzeichnet, daß man Mineralöle mit einem Siedepunkt von 400 bis 550°C verwendet.

4. Verfahren nach den Ansprüchen 1 bis 3, dadurch gekennzeichnet, daß man je Mol Adipinsäure 3 bis 15 Mol Ammoniak anwendet.

5. Verfahren nach den Ansprüchen 1 bis 4, dadurch gekennzeichnet, daß man überschüssiges Ammoniak im Kreis führt.

6. Verfahren nach den Ansprüchen 1 bis 5, dadurch gekennzeichnet, daß man Phosphorsäure, Phosphorsäureester oder Ammoniumsalze der Phosphorsäure als Katalysatoren mitverwendet.

7. Verfahren nach den Ansprüchen 1 bis 6, dadurch gekennzeichnet, daß man 0,1 bis 2 kg Adipinsäure je Stunde und Liter Reaktionsraum zuführt.

8. Verfahren nach den Ansprüchen 1 bis 7, dadurch gekennzeichnet, daß man Adipodinitril in dem Maße wie es entsteht aus dem Reaktionsgemisch austrägt.

9. Verfahren nach den Ansprüchen 1 bis 8, dadurch gekennzeichnet, daß man einen Teil des schwerflüchtige Nebenprodukte enthaltenden Verdünnungsmittels ausschleust und durch frisches Verdünnungsmittel ersetzt.

## Claims

1. A process for the preparation of adipodinitrile by reacting adipic acid with excess ammonia at from 250 to 350°C in the liquid phase in the presence of a diluent, wherein a liquid hydrocarbon which is inert under the reaction conditions is used as the diluent.

2. A process as claimed in claim 1, wherein a mineral oil having a boiling point of at least 350°C is used.

3. A process as claimed in claims 1 and 2, wherein a mineral oil having a boiling point of from 400 to 550°C is used.

4. A process as claimed in claims 1 to 3, wherein from 3 to 15 moles of ammonia are used per mole of adipic acid.

5. A process as claimed in claims 1 to 4, wherein excess anmonia is recycled.

6. A process as claimed in claims 1 to 5, wherein phosphoric acid, a phosphate or an ammonium salt of phosphoric acid is co-used as catalyst.

7. A process as claimed in claims 1 to 6, wherein adipic acid is introduced in an amount of from 0.1 to 2 kg per hour per liter of reaction space.

8. A process as claimed in claims 1 to 7, wherein the rate of discharge of adipodinitrile from the reaction mixture is equal to the rate of formation of adipodinitrile.

9. A process as claimed in claims 1 to 8, wherein some of the diluent containing sparingly volatile by-products is removed, and replaced by fresh diluent.

## Revendications

1. Procédé de préparation d'adiponitrile par réaction d'acide adipique avec de l'ammoniac en excès, à une température de 250 à 350°C, en phase liquide, avec utilisation de diluants, caractérisé par le fait qu'on utilise, comme diluant, des hydrocarbures inertes liquides dans les conditions de la réaction.

2. Procédé selon la revendication 1, caractérisé par le fait qu'on utilise des huiles minérales d'un point d'ébullition d'au moins 350°C.

3. Procédé selon les revendications 1 et 2, caractérisé par le fait qu'on utilise des huiles minérales d'un point d'ébullition de 400 à 550°C.

4. Procédé selon les revendications 1 à 3, caractérisé par le fait qu'on applique 3 à 15 moles d'ammoniac par mole d'acide adipique.

5. Procédé selon les revendications 1 à 4, caractérisé par le fait qu'on fait circuler en cycle fermé de l'ammoniac en excès.

6. Procédé selon les revendications 1 à 5, caractérisé par le fait qu'on utilise conjointement, comme catalyseurs, de l'acide phosphorique, de l'ester phosphorique ou des sels d'ammonium d'acide phosphorique.

7. Procédé selon les revendications 1 à 6, caractérisé par le fait que l'on amène à la chambre de réaction 0,1 à 2 kg d'acide adipique par heure et par litre.

8. Procédé selon les revendications 1 à 7, caractérisé par le fait que l'adiponitrile est récolté à mesure qu'il se forme à partir du mélange de réaction.

9. Procédé selon les revendications 1 à 8, caractérisé par le fait que l'on évacue une partie du diluant contenant des sous-produits faiblement volatils et on les remplace par du diluant frais.